# EUROPEAN PATENT APPLICATION

(11) **EP 4 756 747 A1**
(43) Date of publication of application: **10.06.2026**
(21) Application number: 25220130.6
(22) Date of filing: 02.12.2025
(51) Int. Cl.: G06V 40/18

(54) **IRIS OUTLINE ESTIMATION**

(30) Priority: 05.12.2024 SE 2430633
(71) Applicant: Tobii AB, 182 17 Danderyd (SE)
(72) Inventor: Ly, Adam, 18217 Danderyd (SE)

(57) **Abstract**

A method for detecting an iris in an image of an eye of a user captured by a camera of an eye tracking system, the method comprising: processing the image to estimate a pupil position in a 3D coordinate system; estimating an approximate optical axis in the 3D coordinate system; estimating an iris centre in the 3D coordinate system based on the pupil position in the 3D coordinate system; and estimating an iris outline in the 3D coordinate system as a circle lying in a plane with a normal parallel to the approximate optical axis, centred at the iris centre with an estimated iris diameter; and estimating an image iris outline by transforming the iris outline in the 3D coordinate system to an image coordinate system.

## Description

### Field

The present disclosure relates to a method for detecting an iris in an image, a method of detecting eyelids in an image and apparatus for performing the same.

### Background

In eye tracking applications, digital images are retrieved of the eyes of a user and the digital images are analysed in order to estimate gaze direction of the user. The estimation of the gaze direction may be based on computer-based image analysis of features of the imaged eye. One known example method of eye tracking includes the use of infrared light and an image sensor. The infrared light is directed towards eye(s) of a user and the reflection of the light is captured by an image sensor.

Portable or wearable eye tracking devices have been previously described. One such eye tracking system is described in U.S. Pat. No. 9,041,787 and PCT patent publication number WO 2019/158709 (which are hereby incorporated by reference in their entirety). A wearable eye tracking device is described using illuminators and cameras for determining gaze direction.

The goal of gaze estimation is to find the gaze axis (or visual axis) of an eye. The gaze axis may be defined as the line through the fovea (a point on the retina in the back of the eye) and the cornea centre of curvature (which is the optical centre of the eye). In PCCR (pupil centre - corneal reflection) methods, the locations of the corneal centre and the pupil centre are found using illuminators, cameras and image processing. The line passing through the corneal centre and the pupil centre, the pupillary axis, does not coincide with the visual axis, but their relation (defined by a foveal offset) can be estimated by a personal calibration procedure where the subject looks at known stimulus points, for example on a display screen. After calibration, for each time frame, an estimate of the pupillary axis can be transformed into an estimate of the visual axis. In practice, however, the relation between the pupillary and visual axes is not entirely fixed for each subject but varies as a consequence of a phenomenon called pupil decentration: when the pupil dilates or contracts, such as when an environment illumination level changes, its centre moves slightly in relation to the rest of the eye. Disclosed systems and methods may address this degradation of calibration in PCCR.

### Summary

According to a first aspect of the present disclosure there is provided a method for detecting an iris in an image of an eye of a user captured by a camera of an eye tracking system, the method comprising:
processing the image to estimate a pupil position in a 3D coordinate system;
estimating an approximate optical axis in the 3D coordinate system;
estimating an iris centre in the 3D coordinate system based on the pupil position in the 3D coordinate system; and
estimating an iris outline in the 3D coordinate system as a circle lying in a plane with a normal parallel to the approximate optical axis, centred at the iris centre with an estimated iris diameter; and
estimating an image iris outline by transforming the iris outline in the 3D coordinate system to an image coordinate system.

Estimating the approximate optical axis in the 3D coordinate system may comprise:
estimating the approximate optical axis as a predicted gaze direction of a previous image; or
estimating the approximate optical axis by processing the image using a pupil centre corneal reflection technique.

The approximate optical may comprises: the pupillary axis; or an axis between a sclera centre and a pupil centre.

Estimating the iris centre in the 3D coordinate system may comprise: estimating the iris centre as the pupil position; or estimating the iris centre using the pupil position and a pupil-centre offset.

Transforming the iris outline in the 3D coordinate system to an image coordinate system may comprise:
determining a cone in the 3D coordinate system having an apex at a position of the camera and a conic section corresponding to the estimated iris outline in the 3D coordinate system; and
determining an intersection of the cone with an image plane of the camera to estimate the image iris outline.

The pupil-centre offset may calculated using a previous image.

The method may comprise refining the estimated image iris outline by performing an iris ellipse fitting process on the image comprising the estimated image iris outline.

The iris ellipse fitting process may comprise:
identifying image intensity edge points within a threshold distance of the estimated image iris outline;
obtaining an eyelid outline by performing an eyelid detection process;
filtering the image intensity edge points using the eyelid outline; and
determining a refined image iris outline by fitting an ellipse to the filtered image intensity edge points.

The method may comprise returning a null result if the number of filtered image points is less than a detection confidence threshold.

Obtaining the eyelid outline by performing an eyelid detection process may comprise:
processing the image to estimate, in an image coordinate system, height extremity points comprising:
   a maximum height point of an upper eyelid; and
   a minimum height point of a lower eyelid;
transforming the height extremity points to a 3D coordinate system;
obtaining a sclera centre, a sclera diameter and a sclera-sclera axis of the user in the 3D coordinate system;
determining an eye coordinate system using the sclera centre, the sclera-sclera axis and the height extremity points;
obtaining a first eye corner position and a second eye corner position in the eye coordinate system;
for each of the upper eyelid and the lower eyelid:
   determining an eyelid plane in the eye coordinate system comprising the first eye corner position, the second eye corner position and the respective height extremity point of the eyelid; and
   estimating an eyelid outline in the eye coordinate system as an intersection between the eyelid plane and a sclera sphere having the sclera centre and the sclera diameter; and
   estimating an image eyelid outline by transforming the eyelid outline from the eye coordinate system to an image coordinate system.

Obtaining the eyelid outline by performing an eyelid detection process may comprise any embodiment of the second aspect.

According to a second aspect of the present disclosure there is provided a method for detecting an upper eyelid and a lower eyelid in an image of an eye of a user captured by a camera of an eye tracking system, the method comprising:
processing the image to estimate, in an image coordinate system, height extremity points comprising:
   a maximum height point of the upper eyelid; and
   a minimum height point of the lower eyelid;
transforming the height extremity points to a 3D coordinate system;
obtaining a sclera centre, a sclera diameter and a sclera-sclera axis of the user in the 3D coordinate system;
determining an eye coordinate system using the sclera centre, the sclera-sclera axis and the height extremity points;
obtaining a first eye corner position and a second eye corner position in the eye coordinate system;
for each of the upper eyelid and the lower eyelid:
   determining an eyelid plane in the eye coordinate system comprising the first eye corner position, the second eye corner position and the respective height extremity point of the eyelid; and
   estimating an eyelid outline in the eye coordinate system as an intersection between the eyelid plane and a sclera sphere having the sclera centre and the sclera diameter; and
   estimating an image eyelid outline by transforming the eyelid outline from the eye coordinate system to an image coordinate system.

Transforming each eyelid outline from the eye coordinate system to an image coordinate system may comprise:
transforming the eyelid outline in the eye coordinate system to an eyelid outline in the 3D coordinate system;
determining a cone in the 3D coordinate system having an apex at a position of the camera and a conic section corresponding to the eyelid outline in the 3D coordinate system; and
determining an intersection of the cone with an image plane of the camera to estimate the image eyelid outline.

Processing the image to estimate each height extremity point of the eyelid in the image coordinate system may comprise:
processing the image with an eye-openness algorithm to estimate the height extremity point.

The eye-openness algorithm may comprise: detecting a largest circle that fits between eyelids calculation; or detecting a largest orthogonal distance between eyelids calculation.

Obtaining the sclera-sclera axis may comprise obtaining a first sclera centre of a first eye of the user and obtaining a second sclera centre of a second eye of the user and defining the sclera-sclera axis as a vector from the first sclera centre to the second sclera centre, in the 3D coordinate system. Determining the eye coordinate system may comprise:
determining a first axis of the eye coordinate system as the sclera-sclera axis;
determining a second axis of the eye coordinate system as a cross product between the first axis and a vector from the first sclera centre to a midpoint between the height extremity points in the 3D coordinate system; and
determining a third axis of the eye coordinate system as a cross product of the first axis and the second axis.

According to a third aspect of the present disclosure there is provided a method for detecting an iris in an image of an eye of a user captured by a camera of an eye tracking system, the method comprising:
determining an approximate image iris outline;
sampling image points within a threshold distance of the image iris outline to identify image points on a gradient contour;
determining an image eyelid outline according to any method for detecting an eyelid outlined described herein;
filtering the identified image points using the image eyelid outline; and
determining a refined image iris outline by fitting an ellipse to the filtered image points.

Determining an approximate image iris outline may comprise any method for detecting an image iris outlined disclosed herein.

According to a fourth aspect of the present disclosure, there is provided an eye tracking system comprising a camera and one or more processors configured to carry out any method described herein.

The eye tracking system may comprise a head-mounted eye tracking system or a remote eye-tracking system.

According to a fifth aspect of the present disclosure there is provided an eye tracking system for detecting an iris in an image of an eye of a user captured by a camera of an eye tracking system, the eye tracking comprising a camera and one or more processors configured to:
process the image to estimate a pupil position in a 3D coordinate system;
estimate an approximate optical axis in the 3D coordinate system;
estimate an iris centre in the 3D coordinate system based on the pupil position in the 3D coordinate system; and
estimate an iris outline in the 3D coordinate system as a circle lying in a plane with a normal parallel to the approximate optical axis, centred at the iris centre with an estimated iris diameter; and
estimate an image iris outline by transforming the iris outline in the 3D coordinate system to an image coordinate system.

According to a sixth aspect of the present disclosure there is provided an eye tracking system for detecting an upper eyelid and a lower eyelid in an image of an eye of a user captured by a camera of an eye tracking system, the eye tracking comprising a camera and one or more processors configured to:
process the image to estimate, in an image coordinate system, height extremity points comprising:
   a maximum height point of the upper eyelid; and
   a minimum height point of the lower eyelid;
transform the height extremity points to a 3D coordinate system;
obtain a sclera centre, a sclera diameter and a sclera-sclera axis of the user in the 3D coordinate system;
determine an eye coordinate system using the sclera centre, the sclera-sclera axis and the height extremity points;
obtain a first eye corner position and a second eye corner position in the eye coordinate system;
for each of the upper eyelid and the lower eyelid:
   determine an eyelid plane in the eye coordinate system comprising the first eye corner position, the second eye corner position and the respective height extremity point of the eyelid; and
   estimate an eyelid outline in the eye coordinate system as an intersection between the eyelid plane and a sclera sphere having the sclera centre and the sclera diameter; and
estimate an image eyelid outline by transforming the eyelid outline from the eye coordinate system to an image coordinate system.

There may be provided a computer program, which when run on a computer, causes the computer to configure any apparatus, including a circuit, controller, converter, or device disclosed herein or perform any method disclosed herein. The computer program may be a software implementation, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software may be an assembly program.

The computer program may be provided on a computer readable medium, which may be a physical computer readable medium such as a disc or a memory device, or may be embodied as a transient signal. Such a transient signal may be a network download, including an internet download. There may be provided one or more non-transitory computer-readable storage media storing computer-executable instructions that, when executed by a computing system, causes the computing system to perform any method disclosed herein.

### Brief Description of the Drawings

One or more embodiments will now be described by way of example only with reference to the accompanying drawings in which:
Figure 1 shows a simplified view of an eye tracking;
Figure 2 shows a simplified example of an image of a pair of eyes, captured by an eye tracking system such as the system of Figure 1;
Figure 3 illustrates a cross-section of an example eye;
Figure 4 illustrates a method of detecting an iris outline in an image of an eye of a user, captured by a camera of an eye tracking system, according to an embodiment of the present disclosure;
Figure 5 illustrates a method for detecting a rough image iris outline in an image of an eye of a user, captured by a camera of an eye tracking system, according to an embodiment of the present disclosure;
Figure 6 illustrates an eye in a 3D coordinate system;
Figure 7 illustrates a method for detecting an upper eyelid and a lower eyelid in an image of an eye of a user captured by a camera of an eye tracking system according to an embodiment of the present disclosure;
Figure 8 illustrates an eye in a 3D coordinate system;
Figure 9 illustrates the results of an eye-openness algorithm showing the largest circle and the height extremity points;
Figure 10A illustrates a rough iris image outline estimated using the method of Figures 5 and 6;
Figure 10B illustrates the image intensity edge points identified by the second step of the method of Figure 4;
Figure 10C illustrates the image upper eyelid outline and image lower eyelid outline estimated using the method of Figure 7; and
Figure 10D illustrates the refined image iris outline obtained by fitting an ellipse to the filtered image intensity edge points according to the fifth step of Figure 5.

### Detailed Description

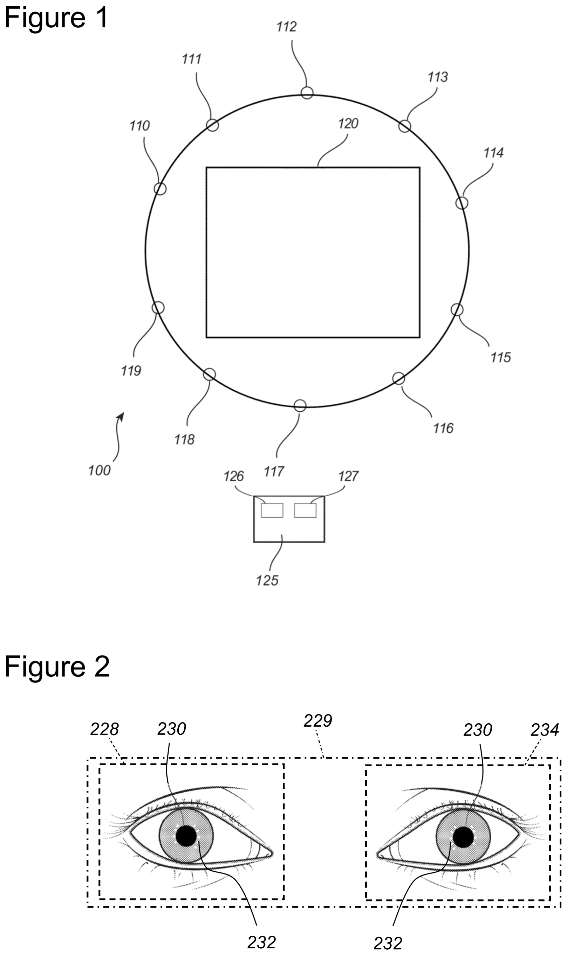

Figure 1 shows a simplified view of an eye tracking system 100 (which may also be referred to as a gaze tracking system) in a head-mounted device in the form of a virtual or augmented reality (VR or AR) device or VR or AR glasses or anything related, such as extended reality (XR) or mixed reality (MR) headsets. The system 100 comprises an image sensor 120 (e.g., a camera) for capturing images of the eyes of the user. The system may optionally include one or more illuminators 110-119 (also referred to herein as light sources) for illuminating the eyes of a user, which may for example be light emitting diodes (LEDs) emitting light in the infrared frequency band, or in the near infrared frequency band and which may be physically arranged in a variety of configurations. The image sensor 120 may for example be an image sensor of any type, such as a complementary metal oxide semiconductor (CMOS) image sensor or a charged coupled device (CCD) image sensor. The image sensor may consist of an integrated circuit containing an array of pixel sensors, each pixel containing a photodetector and an active amplifier. The image sensor may be capable of converting light into digital signals. In one or more examples, it could be an infrared image sensor or IR image sensor, an RGB sensor, an RGBW sensor or an RGB or RGBW sensor with IR filter.

The eye tracking system 100 may comprise circuitry or one or more controllers 125, for example including a receiver 126 and processing circuitry 127, for receiving and processing the images captured by the image sensor 120. The circuitry 125 may for example be connected to the image sensor 120 and the optional one or more illuminators 110-119 via a wired or a wireless connection and be co-located with the image sensor 120 and the one or more illuminators 110-119 or located at a distance, e.g., in a different device. In another example, the circuitry 125 may be provided in one or more stacked layers below the light sensitive surface of the light sensor 120.

The eye tracking system 100 may include a display (not shown) for presenting information and/or visual stimuli to the user. The display may comprise a VR display which presents imagery and substantially blocks the user's view of the real-world or an AR display which presents imagery that is to be perceived as overlaid over the user's view of the real-world.

The location of the image sensor 120 for one eye in such a system 100 is generally away from the line of sight for the user in order not to obscure the display for that eye. This configuration may be, for example, enabled by means of so-called hot mirrors which reflect a portion of the light and allows the rest of the light to pass, e.g., infrared light is reflected, and visible light is allowed to pass.

While in the above example the images of the user's eye are captured by a head-mounted image sensor 120, in other examples the images may be captured by an image sensor that is not head-mounted. Such a non-head-mounted system may be referred to as a remote system.

In an eye tracking system, a gaze signal can be computed for each eye of the user (left and right). The quality of these gaze signals can be reduced by disturbances in the input images (such as image noise) and by incorrect algorithm behaviour (such as incorrect predictions). A goal of the eye tracking system is to deliver a gaze signal that is as good as possible, both in terms of accuracy (bias error) and precision (variance error). For many applications it can be sufficient to deliver only one gaze signal per time instance, rather than both the gaze of the left and right eyes individually. Further, the combined gaze signal can be provided in combination with the left and right signals. Such a gaze signal can be referred to as a combined gaze signal.

Figure 2 shows a simplified example of an image 229 of a pair of eyes, captured by an eye tracking system such as the system of Figure 1. The image 229 can be considered as including a right-eye-image 228, of a person's right eye, and a left-eye-image 234, of the person's left eye. In this example the right-eye-image 228 and the left-eye-image 234 are both parts of a larger image of both of the person's eyes. In other examples, separate image sensors may be used to acquire the right-eye-image 228 and the left-eye-image 234. In other examples, multiple image sensors may be used to acquire images capturing both eyes.

The system may employ image processing (such as digital image processing) for extracting features in the image. The system may for example identify a position of the pupil 230 in the one or more images captured by the image sensor. The system may determine the position of the pupil 230 using a pupil detection process. The system may also identify corneal reflections (also known as glints) 232 located in close proximity to the pupil 230. The system may estimate a corneal centre and / or a distance to the user's eye based on the corneal reflections 232. For example, the system may match each of the individual corneal reflections 232 for each eye with a corresponding illuminator and determine the corneal centre of each eye and / or the distance to the user's eye based on the matching. To a first approximation, the eye tracking system may approximate the gaze axis of the eye of the user based on the pupillary axis - a vector passing through a centre of the pupil 230 and the corneal centre. The direction of gaze corresponds to the axis from the fovea of the eye through the corneal centre. The angle between the pupillary axis and the gaze direction is the *foveal offset,* which typically varies from user to user and is in the range of a few degrees. The eye tracking system may perform a calibration procedure, instructing the user to gaze in a series of predetermined directions (e.g., via instructions on a screen), to determine the foveal offset.

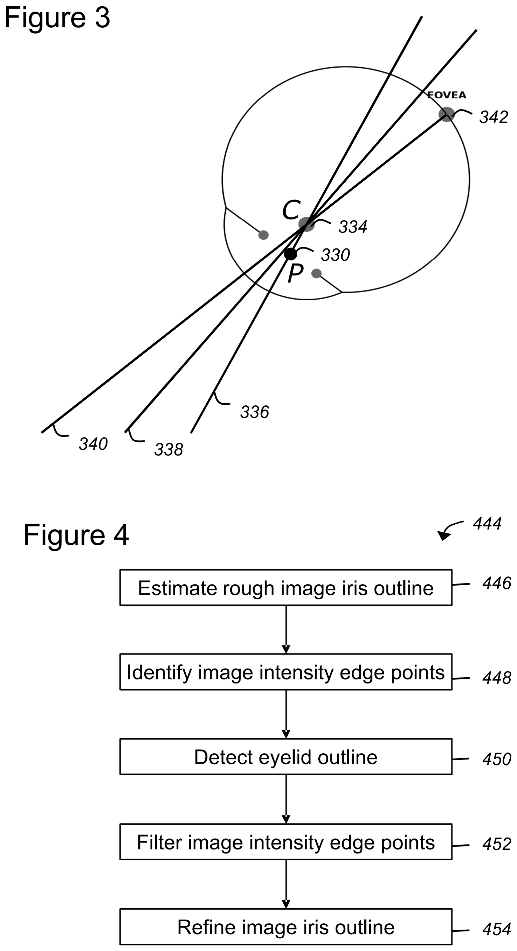

Figure 3 illustrates a cross-section of an example eye. The gaze axis (or visual axis) 340 of an eye corresponds to the line through the fovea 342 (a point on the retina in the back of the eye) and the cornea centre of curvature, C, 334 (which is the optical centre of the eye). In PCCR (pupil centre - corneal reflection) the locations of the corneal centre, C, 334 and the pupil centre, P, 330, are found using illuminators, cameras and image processing. The line passing through the corneal centre, C, 334 and the pupil centre, P, 330 is the pupillary axis 336. The pupillary axis 336 does not coincide with the visual axis 340, but their relation can be estimated by a personal calibration procedure where the subject looks at known stimulus points, for example on a display screen. After calibration, for each time frame, an estimate of the pupillary axis 336 can be transformed into an estimate of the gaze axis 340.

In practice, however, the relation between the pupillary axis 336 and the gaze axis 340 (foveal offset) is not entirely fixed for each subject but varies with time as a consequence of a phenomenon called pupil decentration: when the pupil dilates or contracts, its centre moves slightly in relation to the rest of the eye. The calibration mapping between the pupillary axis and visual axis therefore degrades as the pupil contracts or dilates due to a change in lighting/illumination level, and this calibration error can be particularly acute when the lighting level is significantly different to the light level used for calibration.

However, the gaze axis 340 and the optical axis 338 which corresponds to a line through the corneal centre, C, 334 and a centre of the iris, have a fixed geometrical relationship for a user (fixed here refers to a fixed geometrical relationship on the timescales relevant to eye tracking, however the relationship may vary over much longer timescales as the user ages). An eye tracking system can detect the optical axis 338 by determining an iris centre from image processing. However, iris detection is considerably more difficult than pupil detection because the contrast is higher at the pupil edge than at the iris edge and the iris is often occluded by eyelids. Therefore, PCCR has typically been used for gaze determination in eye tracking systems.

In practice, iris detection may be used to complement PCCR because the precision and trackability of iris detection is typically inferior to pupil detection. For example, iris detections with high measurement confidence can be used to track a pupil-centre offset. A pupil-centre offset is the offset between the iris centre and the pupil centre. By tracking the pupil-centre offset, pupil decentration can be corrected for. For example, the evolving pupil-centre offset may be used to update a transformational mapping between the pupillary axis and the visual axis. The pupil-centre offset varies on a slow timescale. Therefore, accurate iris detections are not required for every captured image.

As described herein, the disclosed methods and systems may include determining positions of eye features, such as pupil position, iris position, corneal centre, sclera centre, gaze axis, pupillary axis and optical axis, in a three-dimensional (3D) coordinate system. The 3D coordinate system may correspond to a coordinate system of a camera that captures the image or of the eye tracking system more generally. The 3D positions of some of the eye features, such as the corneal centre and sclera centre, may be determined using back-projection (and some additional geometry) from two or more images of the same eye captured at substantially the same time point. The two more images may be captured by separate cameras with known positions in the 3D coordinate system or may be captured by the same camera but with different illuminators lit and reflected in the cornea. In virtual reality systems, one image of the eye with several lit illuminators can suffice for determining the corneal centre. Such techniques are known in the art and not described further here.

Figure 4 illustrates a method 444 of detecting an iris outline in an image of an eye of a user, captured by a camera of an eye tracking system, according to an embodiment of the present disclosure.

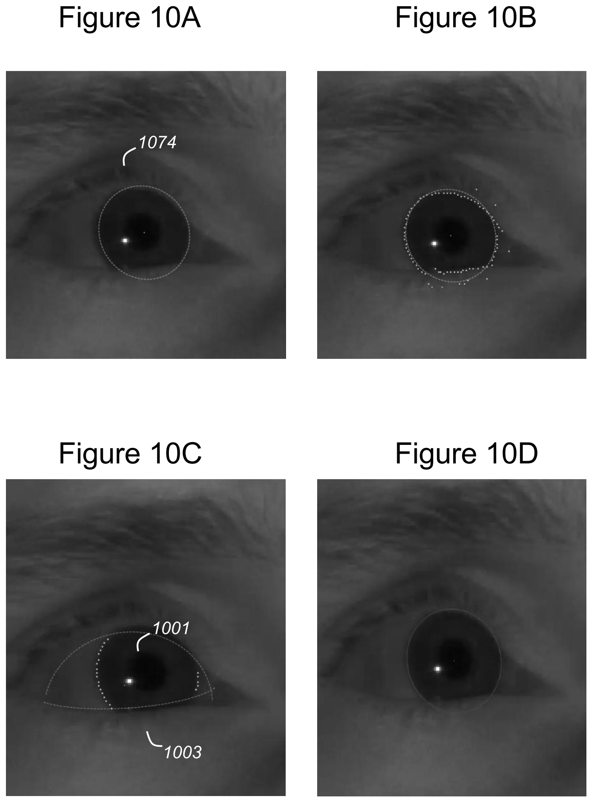

A first step 446 comprises estimating a rough or approximate image iris outline. As described herein, an image iris outline comprises an outline of the iris in an image of the eye. Figure 10A illustrates a rough iris image outline as estimated by an embodiment method for performing the first step 446, described further below.

A second step 448 comprises identifying image intensity edge points within a threshold distance of the estimated image iris outline. Image intensity edge points correspond to image points at an image location with a sharp intensity contrast (and within a threshold distance of the approximate image iris outline). In some examples, the second step 448 may identify the image intensity edge points by sampling image points within the threshold distance of the estimated image iris outline and identifying intensity maxima in a radial direction (from the iris centre). This results in a number of points on the rough iris outline (see Figure 10B). In some examples, the second step 448 may identify image intensity edge points using an edge detection method. The edge detection method may identify image intensity edge points corresponding to an edge of the iris, eyelid or eyelash (see Figure 10B), which tend to form regions of high contrast. In this way, the image intensity edge points may be considered as candidate iris outline points. Known edge detection techniques such as intensity gradient technique and Canny edge detection may be used to identify the image intensity edge points.

A third step 450 comprises obtaining an eyelid outline by performing an eyelid detection process. An eyelid detection method for performing the third step 450 is described in detail below.

A fourth step 452 comprises filtering the image intensity edge points using the eyelid outline. Figure 10C illustrates filtered image intensity edge points that have been filtered by an eyelid outline.

A fifth step 454 comprises determining a refined image outline by fitting an ellipse to the filtered image intensity edge points (see Figure 10D). The fifth step may be performed using a known curve fitting technique. In some examples, the method 444 may comprise returning a null result for the image iris outline if the number of filtered image points is less than a detection confidence threshold. In other words, the method may return a "iris detection confidence too low" or similar output if there are too few filtered image points for fitting an ellipse with confidence.

For the first step 446, the rough image iris outline estimation may comprise performing a machine learning (ML) analysis of the image to directly detect the image iris outline. For example, a gradient boosted tree algorithm may be used to regress the position of the iris (image iris outline). However, such an approach is time and resource intensive and can have similar computational complexity to pupil detection methods. Therefore, detecting irises as a complement to PCCR can double the computational requirements. ML approaches also require training data to train the algorithm. Annotating such training data is a difficult task irrespective of whether human or automatic annotation methods are employed.

For the third step 450, the eyelid may be detected by identifying multiple points on the eyelid edge together with model fitting. However, identifying ground truth for such landmarks can be difficult because some landmarks are not strictly defined.

The present disclosure includes systems and methods that can provide: (i) an improved first step 446 of rough image iris outline estimation; (ii) an improved third step 450 of eyelid detection; and (iii) an improved method 444 of iris detection utilising one or both of (i) and (ii).

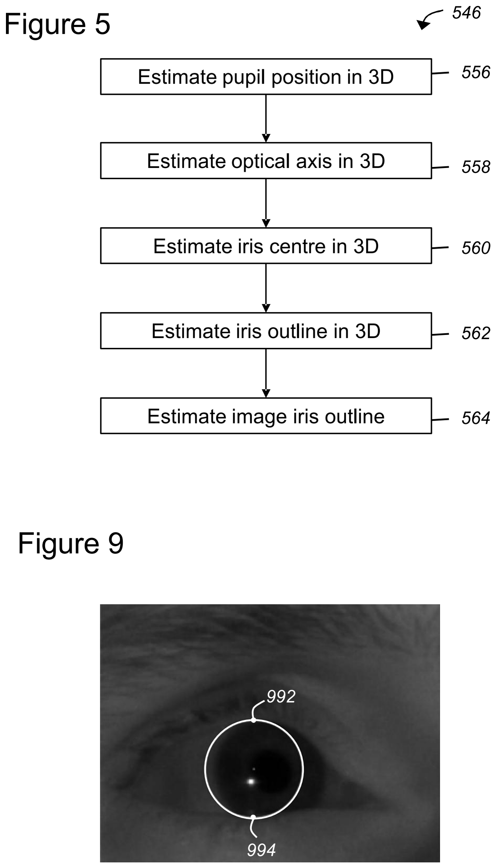

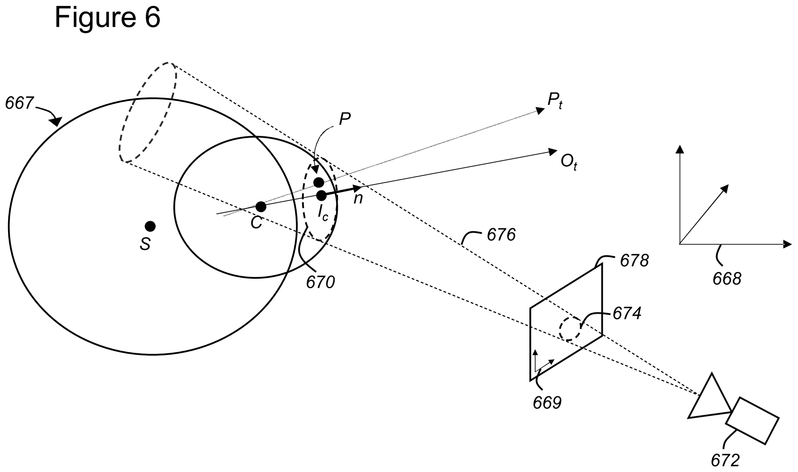

Figure 5 illustrates a method 546 for detecting a (rough) image iris outline in an image of an eye of a user, captured by a camera of an eye tracking system, according to an embodiment of the present disclosure. The reference number 546 is used to correspond to the first step 446 of the method of Figure 4. The method 546 is described with reference to Figure 6 which illustrates the eye 667 in a 3D coordinate system 668.

A first step 556 comprises processing the image to estimate a pupil position, P, in the 3D coordinate system 668. As defined herein, the pupil position, P, may comprise a pupil centre. The 3D coordinate system 668 may comprise a global coordinate system such as a camera coordinate system, with a position of the camera 672 at the origin. The first step 556 may comprise: (i) processing the image to estimate a pupil position in an image coordinate system 669 (a 2D coordinate system corresponding to axes of the image); and (ii) transforming the pupil position from the image coordinate system 669 to the 3D coordinate system 668. The first step 556 may be performed using known techniques, for example the pupil detection component of PCCR. The pupil position may be detected in the image coordinate system 669 using a known technique such as a gradient boosted tree algorithm. The pupil position may be transformed from the image coordinate system 669 to the 3D coordinate system 668 using a known technique such as back-projection. For example, back-projection can provide a pupil ray in the 3D coordinate system 668. The pupil position can be obtained by intersecting the pupil ray with a pupil sphere. In some examples, the pupil sphere may be approximated as the sclera sphere. In more precise examples, the pupil sphere may be estimated as having the sclera centre and a hard-coded sclera-pupil radius (which is slightly less than the sclera radius), corresponding to the distance from the sclera centre to the pupil centre.

A second step 558 comprises estimating an approximate or rough optical axis, Ot, in the 3D coordinate system 668. In some examples, the method may comprise obtaining the approximate optical axis, Oₜ, as a gaze direction (visual axis) or a pupillary axis determined for a previous image using PCCR. In some examples, the method may comprise approximating the optical axis, Ot, as a visual axis or a pupillary axis, Pt, determined by processing the (current) image using PCCR. In some examples, the optical axis, Oₜ, may be estimated as an axis through the sclera centre, S, and the pupil centre, P. In this example, the optical axis, Ot, is illustrated as offset from the pupillary axis, Pt, - a line passing through the corneal centre, C, (which may also be calculated via PCCR) and the pupil position, P.

A third step 560 comprises estimating an iris centre, I_{c}, in the 3D coordinate position 668 based on the pupil position, P, in the 3D coordinate system 668. In the simplest example, the iris centre, I_{c}, may be estimated as the pupil position, P. In some examples, the iris centre, I_{c}, may be estimated using the pupil position and an estimate of the pupil-centre offset. The pupil-centre offset is the offset (or vector) between the pupil centre, P, and the iris centre, I_{c}. The estimate of the pupil-centre offset may comprise a value of the pupil-centre offset calculated for a previous image. The pupil-centre offset is a measure of the pupil decentration and varies slowly. Therefore, using a pupil-centre offset calculated for a previous image will provide a sufficient estimate.

A fourth step 562 comprises estimating an iris outline 670 in the 3D coordinate system 668 as a circle lying in a plane with a normal, n, parallel to the approximate optical axis, Oₜ, and centred at the iris centre, I_{c}, with an estimated iris diameter. The estimated iris diameter may comprise an iris diameter calculated for a previous image calculated during a personal calibration process or estimated based on a population average.

A fifth step 564 comprises estimating an image iris outline 674 by transforming the iris outline 670 in the 3D coordinate system 668 to the image coordinate system 669. In this example, transforming the iris outline 670 to the image coordinate system 669 uses a conical transformation technique.

The conical transformation technique may comprise: defining a cone 676 in the 3D coordinate system 668 having an apex at a position of the camera 672 and a conic section equal to the iris outline 670 in the 3D coordinate system 668; and estimating the image iris outline 674 as an intersection of the cone 676 with an image plane 678 of the camera 672.

As the camera 672 is off-axis from the optical axis, the image iris outline will be an ellipse.

In other examples, a different transformation technique may be used to transform the iris outline 670 to the image coordinate system 669. For example, a cylindrical transformation may be used comprising: defining a cylinder in the 3D coordinate system 668 with a cross-section corresponding to the iris outline 670 and an axis parallel to the normal, n; estimating an iris shape based on an intersection of the cylinder with the image plane 678 of the camera 672; and scaling the iris shape by a scaling factor to estimate the image iris outline 674.

The iris shape calculated with the cylindrical transformation is limited to the half axes and the rotation of the ellipse - the size (scale) of the image iris outline is still unknown. The scaling factor and size of the iris may be estimated using the estimated iris diameter from a previous image.

Returning to Figure 4, following the fifth step 454, the method 444 may comprise transforming the refined image iris outline back to the 3D coordinate system 668. The method may then determine a value of the iris diameter and a value of the pupil-centre offset (by calculating the offset between the pupil centre, P, and the iris centre, I_{c}). For a subsequent image, the method 546 of Figure 5 may use the stored values of the pupil-centre offset and/or the iris diameter in the respective third and/or fourth steps 560, 562. For a first image (when no prior image values exist), the estimate of the iris diameter and/or the pupil-centre offset may comprise a respective population average or be determined with a different technique (such as direct detection of the iris in the image). The iris diameter is constant and does not vary for a person. Therefore, the method may comprise determining an average value of estimated iris diameters over a plurality of images as a way to provide a more accurate estimate of the iris diameter.

The method 564 of Figure 5 can advantageously be computed with a few floating-point operations and therefore drastically reduces the computation complexity relative to a machine learning approach such as a boosted tree algorithm. A further advantage of the method 564 is that no training data is required. A yet further advantage is that the method makes use of eye feature values that are already typically calculated for eye tracking using PCCR, such as the pupil position, P, the corneal centre, C, the sclera centre, S, etc. Therefore, the method 564 of Figure 5 results in minimal additional computational effort for an eye tracking system already employing PCCR.

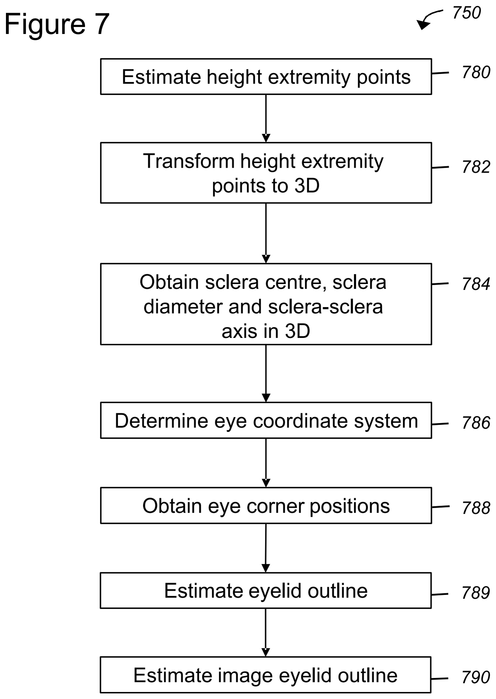

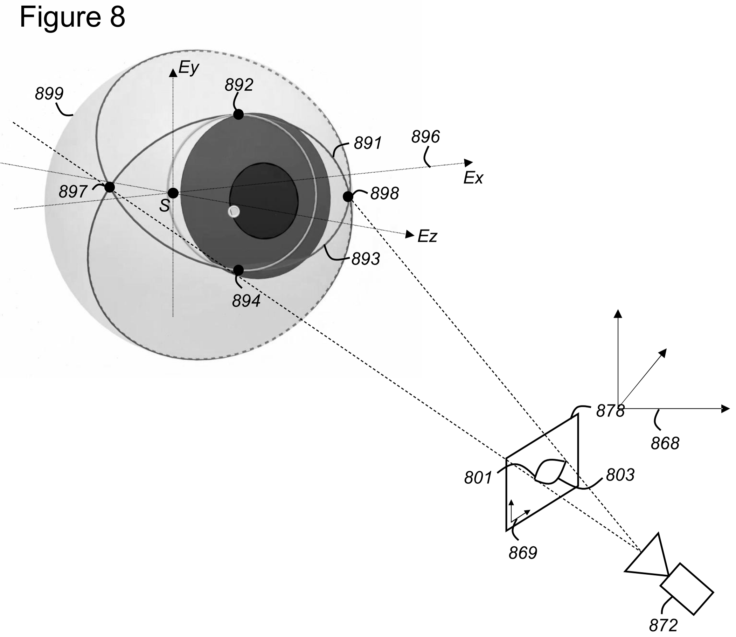

Figure 7 illustrates a method 750 for detecting an upper eyelid and a lower eyelid in an image of an eye of a user captured by a camera of an eye tracking system according to an embodiment of the present disclosure. The reference number 750 is used to correspond to the third step 450 of the method of Figure 4. The method 750 is described with reference to Figure 8 which illustrates the eye 867 in a 3D coordinate system 868 and with reference to Figure 9 which illustrates an image of the eye.

A first step 780 comprises processing the image to estimate height extremity points in an image coordinate system 869. The height extremity points comprise a maximum height point 992 of the upper eyelid and a minimum height point 994 of the lower eyelid. The first step 780 may comprise processing the image with an eye-openness algorithm to estimate the height extremity points. The eye-openness algorithm may comprise a known technique such as a calculation to detect the largest circle that fits between the eyelid. Figure 9 illustrates the results of such a technique showing the largest circle and the height extremity points 992, 994. In some examples, the eye-openness algorithm may comprise a calculation of the longest line which intercepts both eyelids orthogonally.

A second step 782 comprises transforming the height extremity points from the image coordinate system 869 to the 3D coordinate system 868. The transformation may be achieved using back projection. For example, back-projection can provide corresponding extremity point rays in the 3D coordinate system 868. The height extremity points can correspond to the intersection of the extremity point rays with the sclera sphere. The transformation provides the maximum height point 892 of the upper eyelid and the minimum height point 894 of the lower eyelid in the 3D coordinate system 868.

A third step 784 comprises obtaining a sclera centre, S, a sclera diameter and a sclera-sclera axis, Ex, of the user, in the 3D coordinate system 868.

The sclera centre, S, sclera diameter and sclera-sclera axis, Ex, may be obtained using known techniques such as PCCR and back-projection. For example, PCCR can provide the corneal centre by identifying glints from multiple illuminators in the image and using the known location of each illuminator relative to the camera to determine the corneal centre. PCCR may also provide the optical axis based on detecting the pupil centre and calibration data. The sclera centre may be determined using the corneal centre and the optical axis. The sclera centre may be determined as a fixed (hard-coded) distance from the corneal centre along the direction of the optical axis. The sclera diameter may be hard-coded. Hard-coded values (sclera diameter, sclera centre -pupil centre distance, sclera centre corneal centre distance etc.) may comprise population average values.

Obtaining the sclera-sclera axis, Ex, may comprise: obtaining (e.g. via PCCR) a first sclera centre of a first eye of the user; obtaining a second sclera centre of a second eye of the user; and defining the sclera-sclera axis, Ex, as a vector from the first sclera centre to the second sclera centre. It will be appreciated that the third step 784 may be performed earlier in the method 750, for example before the first step 780 or the second step 782.

A fourth step 786 comprises determining an eye coordinate system 896 using the sclera centre, S, the sclera-sclera axis, Ex, and the height extremity points 892, 894. In this example, a first axis of the eye coordinate system 896 comprises the sclera-sclera axis, Ex. A second axis, Ey, of the eye coordinate system 896 comprises a cross-product between the first axis, Ex, and a vector from the sclera centre, S, to a midpoint between the maximum height point 892 of the upper eyelid and the minimum height point 894 of the lower eyelid. A third axis, Ez, comprises a cross product of the first axis, Ex, and the second axis, Ey.

A fifth step 788 comprises obtaining a first eye corner position 897 and a second eye corner position 898 in the eye coordinate system 896. The first and second eye corner positions 897, 898 are the corners of the eye where the eyelids meet and at the apex of the visible part of the sclera. An advantage of defining the eye coordinate system 896 is that the first and second eye coordinate positions 897, 898 have fixed positions within the eye coordinate system 896. In other words, the first and second eye corner positions 897, 898 in the eye coordinate system 896 are insensitive to the user's head rotation and eye openness. As the eye openness varies, the second axis, Ey, and the third axis, Ez, may vary via a minor rotation about the first axis. However, the first and second eye corner positions 897, 898 will be unaffected. In this way, the first and second eye corner positions 897, 898 in the eye coordinate system 896 provide robust landmarks for eyelid detection.

As the eye corner positions 897, 898 are fixed within the eye coordinate system 896, they may only need to be calculated once. Therefore, the fifth step 788 may comprise obtaining the first and second eye corner positions 897, 898 from a memory /storage. To generate and store the first and second eye corner positions 897, 898 in the eye coordinate system initially, the first and second eye corner positions 897, 898 may be directly detected in an image coordinate system by processing an image of the eye, transformed to the 3D coordinate system 868 (e.g. using back projection) and then transformed to an eye coordinate system 896. Detection of the corner positions in the image may use a known image processing technique.

A sixth step 789 comprises: determining an upper eyelid plane in the eye coordinate system 896 as a plane comprising the first eye corner position 897, the second eye corner position 898 and the maximum height point 892 of the upper eyelid; determining a lower eyelid plane in the eye coordinate system 896 as a plane comprising the first eye corner position 897, the second eye corner position 898 and the minimum height point 894 of the lower eyelid; estimating an upper eyelid outline 891 in the eye coordinate system 896 as an intersection between the upper eyelid plane and a sclera sphere 899 having the sclera centre, S, and the sclera diameter; and estimating a lower eyelid outline 893 in the eye coordinate system 896 as an intersection between the lower eyelid plane and the sclera sphere 899. The intersection between the upper / lower eyelid plane and the sclera sphere is a circle. In some examples, the upper & lower eyelid outlines 891, 893 may comprise the respective circle. In some examples, the upper & lower eyelid outlines 891, 893 may comprise the arc of the circle between the first and second corner positions 897, 898 and comprising the respective height extremity point 892, 894.

A seventh step 790 comprises: estimating an image upper eyelid outline 801 by transforming the upper eyelid outline 891 from the eye coordinate system 896 to the image coordinate system 869; and estimating an image lower eyelid outline 803 by transforming the lower eyelid outline 893 from the eye coordinate system 896 to the image coordinate system 869. The seventh step 790 may comprise transforming the eyelid outlines 891, 893 from the eye coordinate system 896 to the 3D coordinate system 868; and transforming the eye outlines in the 3D coordinate system 868 to the image coordinate system 869.

The seventh step 790 may use a conical transformation in a similar manner to that described above with respect to Figures 5 and 6. For example, the method may determine an upper eyelid cone having an apex at a position of the camera 872 and a conic section corresponding to the upper eyelid outline 891. Similarly, the method may determine a lower eyelid cone having an apex at a position of the camera 872 and a conic section corresponding to the lower eyelid outline 893. The method may estimate the image upper/lower eyelid outlines 801, 803 by determining the intersection between the image plane of the camera 872 and the respective cone. The transformation of the circular iris outlines 891, 893 in the 3D / eye coordinate systems to the off-axis image coordinate system results in the image eyelid outlines 801, 803 comprising ellipses.

The eyelid detection method of Figure 7 advantageously makes use of the most stable eyelid landmarks - the corner positions. By performing the eyelid detection in the eye coordinate system where the corner positions are stable and fixed, the eye detection algorithm is advantageously robust to head movement and eye openness variation. In contrast, a curve fitting approach performed in the image plane using the same landmarks would be sensitive to head orientation. The eyelid detection method also makes use of already available 3D data values (sclera centre & diameter) adding minimal computational complexity to any eye tracking algorithm.

Although the eyelid detection method of Figure 7 has been described in relation to iris detection (e.g. the third step of Figure 4), the method of eyelid detection is not limited thereto and the eyelid detection method may find uses in other processes for eye feature identification and/or eye tracking.

Figures 10A to 10D illustrate different steps of the method of claim 4 when utilising the rough image iris outline detection of Figures 5 & 6 and the eyelid detection method of Figures 7 to 9.

Figure 10A illustrates the rough iris image outline 1074 estimated using the method of Figure 5. Figure 10B illustrates the image intensity edge points identified by the second step of the method of Figure 4. Figure 10C illustrates the image upper eyelid outline 1001 and image lower eyelid outline 1003 estimated using the method of Figure 7. The image eyelid outlines 1001, 1003 are used to filter the image intensity edge points according to the fourth step of Figure 4. Figure 10D illustrates the refined image iris outline obtained by fitting an ellipse to the filtered image intensity edge points according to the fifth step of Figure 5. An excellent fit to the iris edge can be seen in the image.

Throughout the present specification, the descriptors relating to relative orientation and position, such as "horizontal", "vertical", "top", "bottom" and "side", are used in the sense of the orientation of the eye or eye tracking system as presented in the drawings. However, such descriptors are not intended to be in any way limiting to an intended use of the described or claimed invention.

It will be appreciated that any reference to "close to", "before", "shortly before", "after" "shortly after", "higher than", or "lower than", etc, can refer to the parameter in question being less than or greater than a threshold value, or between two threshold values, depending upon the context.

## Claims

1. A method for detecting an iris in an image of an eye of a user captured by a camera of an eye tracking system, the method comprising:
processing the image to estimate a pupil position in a 3D coordinate system;
estimating an approximate optical axis in the 3D coordinate system;
estimating an iris centre in the 3D coordinate system based on the pupil position in the 3D coordinate system; and
estimating an iris outline in the 3D coordinate system as a circle lying in a plane with a normal parallel to the approximate optical axis, centred at the iris centre with an estimated iris diameter; and
estimating an image iris outline by transforming the iris outline in the 3D coordinate system to an image coordinate system.

2. The method of claim 1 comprising:
refining the estimated image iris outline by performing an iris ellipse fitting process on the image comprising the estimated image iris outline, the iris ellipse fitting process comprising:
identifying image intensity edge points within a threshold distance of the estimated image iris outline;
obtaining an eyelid outline by performing an eyelid detection process;
filtering the image intensity edge points using the eyelid outline; and
determining a refined image iris outline by fitting an ellipse to the filtered image intensity edge points.

3. The method of claim 2, comprising:
Returning a null result if the number of filtered image points is less than a detection confidence threshold.

4. The method of any preceding claim, wherein estimating the approximate optical axis in the 3D coordinate system comprises:
estimating the approximate optical axis as a predicted gaze direction of a previous image; or
estimating the approximate optical axis by processing the image using a pupil centre corneal reflection technique.

5. The method of any preceding claim wherein the approximate optical comprises:
the pupillary axis; or
an axis between a sclera centre and a pupil centre.

6. The method of any preceding claim, wherein estimating the iris centre in the 3D coordinate system comprises:
estimating the iris centre as the pupil position; or
estimating the iris centre using the pupil position and a pupil-centre offset and/or wherein the pupil-centre offset may be calculated using a previous image.

7. The method of any preceding claim, wherein transforming the iris outline in the 3D coordinate system to an image coordinate system comprises:
determining a cone in the 3D coordinate system having an apex at a position of the camera and a conic section corresponding to the estimated iris outline in the 3D coordinate system; and
determining an intersection of the cone with an image plane of the camera to estimate the image iris outline.

8. A method for detecting an upper eyelid and a lower eyelid in an image of an eye of a user captured by a camera of an eye tracking system, the method comprising:
processing the image to estimate, in an image coordinate system, height extremity points comprising:
a maximum height point of the upper eyelid; and
a minimum height point of the lower eyelid;
transforming the height extremity points to a 3D coordinate system;
obtaining a sclera centre, a sclera diameter and a sclera-sclera axis of the user in the 3D coordinate system;
determining an eye coordinate system using the sclera centre, the sclera-sclera axis and the height extremity points;
obtaining a first eye corner position and a second eye corner position in the eye coordinate system;
for each of the upper eyelid and the lower eyelid:
determining an eyelid plane in the eye coordinate system comprising the first eye corner position, the second eye corner position and the respective height extremity point of the eyelid; and
estimating an eyelid outline in the eye coordinate system as an intersection between the eyelid plane and a sclera sphere having the sclera centre and the sclera diameter; and
estimating an image eyelid outline by transforming the eyelid outline from the eye coordinate system to an image coordinate system.

9. The method of claim 8, wherein transforming each eyelid outline from the eye coordinate system to an image coordinate system comprises:
transforming the eyelid outline in the eye coordinate system to an eyelid outline in the 3D coordinate system;
determining a cone in the 3D coordinate system having an apex at a position of the camera and a conic section corresponding to the eyelid outline in the 3D coordinate system; and
determining an intersection of the cone with an image plane of the camera to estimate the image eyelid outline.

10. The method of claim 8 or claim 9, wherein processing the image to estimate each height extremity point of the eyelid in the image coordinate system comprises:
processing the image with an eye-openness algorithm to estimate the height extremity point, and/or wherein the eye-openness algorithm may comprise:
detecting a largest circle that fits between eyelids calculation; or
detecting a largest orthogonal distance between eyelids calculation.

11. The method of any of claims 8 to 10, wherein obtaining the sclera-sclera axis comprises obtaining a first sclera centre of a first eye of the user and obtaining a second sclera centre of a second eye of the user and defining the sclera-sclera axis as a vector from the first sclera centre to the second sclera centre, in the 3D coordinate system, wherein determining the eye coordinate system comprises:
determining a first axis of the eye coordinate system as the sclera-sclera axis;
determining a second axis of the eye coordinate system as a cross product between the first axis and a vector from the first sclera centre to a midpoint between the height extremity points in the 3D coordinate system; and
determining a third axis of the eye coordinate system as a cross product of the first axis and the second axis.

12. A method for detecting an iris in an image of an eye of a user captured by a camera of an eye tracking system, the method comprising:
determining an approximate image iris outline;
sampling image points within a threshold distance of the image iris outline to identify image points on a gradient contour;
determining an image eyelid outline according to any of methods 7 to 10;
filtering the identified image points using the image eyelid outline; and
determining a refined image iris outline by fitting an ellipse to the filtered image points;
and/or wherein determining an approximate image iris outline may comprise the method of any of claims 1 to 7

13. An eye tracking system comprising a camera and one or more processors configured to carry out the method of any preceding claim and/or wherein the eye tracking system may comprise a head-mounted eye tracking system or a remote eye-tracking system.

14. An eye tracking system for detecting an iris in an image of an eye of a user captured by a camera of an eye tracking system, the eye tracking comprising a camera and one or more processors configured to:
process the image to estimate a pupil position in a 3D coordinate system;
estimate an approximate optical axis in the 3D coordinate system;
estimate an iris centre in the 3D coordinate system based on the pupil position in the 3D coordinate system; and
estimate an iris outline in the 3D coordinate system as a circle lying in a plane with a normal parallel to the approximate optical axis, centred at the iris centre with an estimated iris diameter; and
estimate an image iris outline by transforming the iris outline in the 3D coordinate system to an image coordinate system.

15. An eye tracking system for detecting an upper eyelid and a lower eyelid in an image of an eye of a user captured by a camera of an eye tracking system, the eye tracking comprising a camera and one or more processors configured to:
process the image to estimate, in an image coordinate system, height extremity points comprising:
a maximum height point of the upper eyelid; and
a minimum height point of the lower eyelid;
transform the height extremity points to a 3D coordinate system;
obtain a sclera centre, a sclera diameter and a sclera-sclera axis of the user in the 3D coordinate system;
determine an eye coordinate system using the sclera centre, the sclera-sclera axis and the height extremity points;
obtain a first eye corner position and a second eye corner position in the eye coordinate system;
for each of the upper eyelid and the lower eyelid:
determine an eyelid plane in the eye coordinate system comprising the first eye corner position, the second eye corner position and the respective height extremity point of the eyelid; and
estimate an eyelid outline in the eye coordinate system as an intersection between the eyelid plane and a sclera sphere having the sclera centre and the sclera diameter; and
estimate an image eyelid outline by transforming the eyelid outline from the eye coordinate system to an image coordinate system.
